# EUROPEAN PATENT SPECIFICATION

(11) **EP 0 830 082 B1**
(45) Date of publication and mention of the grant of the patent: **03.12.2003**
(21) Application number: 97914848.3
(22) Date of filing: 11.03.1997
(51) Int. Cl.: A61B 10/00, B01L 3/00, G01N 33/543

(54) **DEVICE FOR THE COLLECTION, TESTING AND SHIPMENT OF BODY FLUID SAMPLES**
VORRICHTUNG ZUM SAMMELN, PRÜFEN UND VERSAND VON KÖRPERFLÜSSIGKEITSPROBEN
DISPOSITIF DE PRELEVEMENT, D'ANALYSE ET D'EXPEDITION D'ECHANTILLONS DE LIQUIDE BIOLOGIQUE

(30) Priority: 11.03.1996 US 613487
(43) Date of publication of application: 25.03.1998
(73) Proprietor: American Bio Medica Corporation, Ancramdale, NY 12503 (US)
(72) Inventor: CIPKOWSKI, Stan, Ancramdale, NY 12503 (US)
(74) Representative: Volmer, Johannes Cornelis
(86) International application number: PCT/US97/03347
(87) International publication number: WO 97/033519

(56) References cited:
- EP-A- 0 634 215
- WO-A-94/06940
- WO-A-95/07659
- FR-A- 2 581 194
- US-A- 4 873 193
- US-A- 5 119 830
- US-A- 5 352 410
- US-A- 5 368 583

## Description

### RELATED APPLICATIONS

For purposes of the United States of America, this is a continuation-in-part application of United States Patent Application Serial No. 08/613,487 filed March 11, 1996.

### TECHNICAL FIELD

The present invention relates to a test kit for the collection and testing of urine samples for drugs of abuse and subsequent shipment of the sample, more particularly, to such a test kit having a cup-like container and a test card for indicating visually the presence of particular drugs of abuse.

### BACKGROUND ART

The increased availability and use of drugs of abuse by the general population has caused employers, governmental agencies, sports groups and other organizations to utilize drug screening both as a condition of employment and in order to maintain safety in the work place. Typical drug screening tests are performed for the purpose of quickly identifying on a qualitative basis the presence of drugs in a body fluid which may be urine. A complete analysis of the sample may then be carried out in a laboratory only if the preliminary screening results are positive. More and more such drug screenings are taking place on site or the work place and are generally carried out by testing personnel who are generally not technically trained, such as laboratory technicians. It is thus important that the drug screening procedure is simple but yet reliable. Further, the test apparatus must be such so as to enable the testing personnel to avoid all contact with the fluid specimen which is being tested.

Various forms of devices have been proposed for the collection and taking of body fluids, such as urine, which have proved to be cumbersome in operation since they involve a number of separate steps. Initially, the sample was collected and several additional steps were then required to transfer the urine sample to an analysis device. This multiple step procedure required the manual handling of the specimen through various devices and the use of such transfer devices inevitably caused spills which may result in contamination to the tester and surroundings. In addition, nontechnical personnel who perform the screening tests on urine samples objected to coming into any kind of contact with the urine sample and even the handling of the sample itself.

Many of the known testing devices were rather complex in that they included a container for the specimen, and, subsequently it was necessary to transfer the specimen or at least a portion thereof to another compartment of the container in order to perform the test. This transfer of the specimen required vigorous shaking of the container or turning the container upside down in order to cause the flow of the specimen into a test compartment. It was therefore necessary to make the containers leak proof under such condition and the results was a complicated and expensive container structure.

Further, the containers incorporated the structure by means of which reagent strips were mounted in a test compartment of the container and which structure also enabled the fluid sample to flow into the test compartment into contact with the reagent strips. Such a mounting of the reagent strips further resulted in complicating the structure of the container since it was also necessary that provision be made to view the reagent strips from outside of the container. This was generally achieved by providing a transparent window or some other mounting of the reagent strips so as to be visible to testing personnel.

US-A-5 119 830 discloses an analytical specimen cup having a lid. This lid comprises outer and inner partitions defining a test space. The inner partition is provided with a valve comprised of a frangible portion. By breaking said frangible portion a fluid sample can be introduced from the cup through the valve into the test space. There the fluid sample contacts a chemical test strip which is mounted in the test space. The results of the test are visible through the transparent outer partition. The chemical test strips may be provided in parallel on a test card in the width direction thereof.

### DISCLOSURE OF INVENTION

It is therefore the principal object of the present invention to provide a simplified and inexpensive device for the collection and testing of body fluid samples, particularly urine, for drugs of abuse and subsequent shipment of the sample.

It is an additional object of the present invention to provide such a device which includes a closed container for retaining a urine sample having such a closure structure that test card having a plurality of test strips thereon may be introduced into the container such that the test strips contact the urine sample.

It is a further object of the present invention to provide a test card having a plurality of immunoassay test strips thereon with each strip being responsive to a particular drug of abuse and having a visual endpoint to indicate the presence or absence of a particular drug.

The objects of the present invention are achieved and the disadvantages of the prior art are eliminated by the drug abuse test kit and test card according to the present invention as defined in claims 1 and 4 respectively. The kit may comprise a cup-like transparent container for retaining a urine sample to be tested. The open top of the container has a closure cover or cap and there is a diametrical slit in the cap. The slit is of such a size to accommodate a test card which has a plurality of immunoassay test strips mounted thereon in parallel on one side and each test strip is responsive to a particular drug of abuse. The test card is insertable through the slit so as to have one end immersed in the urine sample to a predetermined depth whereby the visual results of each test strip can be seen through the transparent wall of the container without removing the test card from the container so as to indicate the presence or absence of a particular drug of abuse in the urine sample. If the sample should test "positive" to indicate the presence of a drug in the urine, it is then necessary to send the sample to a certified laboratory for confirmatory testing. For this purpose, a second closure cap which is solid, i.e., not slit, is provided which may be threaded onto the open end of the cup-like container. The test card is removed from the container, the solid closure cap is threaded on to close the container and the container is then ready for shipment to a laboratory.

As described above, the test kit includes a drug abuse test device for collecting and testing a urine sample. This test device comprises a cup-like container having a transparent wall and having an open top upon which is threaded a closure cover provided with a slit therein to receive a test card. A solid second closure cap which threads over the outer end of the cup-like container is provided to seal the container to permit the safe shipment of a fluid sample therein.

The test kit also includes a screen test card for drugs of abuse which may comprise a thin flat member having the size and shape of a business card. A plurality of immunoassay test strips are fastened side by side in parallel on one side of the test card within the outline of the card. Each test strip is reactive to provide a visual indication in response to a particular drug of abuse. This test card thus provides for the simultaneous detection of multiple analytes.

### BRIEF DESCRIPTION OF THE DRAWINGS

Other objects and advantages of the present invention will be apparent upon reference to the accompanying description when taken in conjunction with the following drawings, which are exemplary, wherein;

Fig. 1 is a perspective view of the drug abuse test kit according to the present invention generally showing the container, the test card partially inserted to the testing position in the container through a slit in the cover;

Fig. 2 is an exploded perspective view of the container according to the present invention for collecting and testing a fluid sample and generally showing the container, a cover having a slit covered with a removable adhesive seal and a second solid closure cap;

Fig. 3 is a plan view of the test side of a test card according to the present invention;

Fig. 4'is a plan view of the reverse side of the test card shown in Fig. 3;

Fig. 5 is an end elevational view of the test card shown in Fig. 3;

Fig. 6 is a sectional view taken along the lines VI-VI of Fig. 3;

Fig. 7 is a plan view of the opened two piece test card before it is folded over to form the test card shown in Figs. 3-6;

Fig. 8 is a plan view of the test side of a modification of the test card;

Fig. 9 is a plan view of another modification of the test card;

Fig. 10 is a sectional view taken along the lines IX-IX of Fig. 8;

Fig. 11 is a plan view of the test side of a further modification of the test card;

Fig. 12 is a plan view of the center ply of the test card of Fig. 11 and showing a test strip in a slot thereof;

Fig. 13 is a plan view of the reverse side of the test card of Fig. 11;

Fig. 14 is a sectional view taken along the lines XIII-XIII of Fig. 11.

### MODES FOR CARRYING OUT THE INVENTION

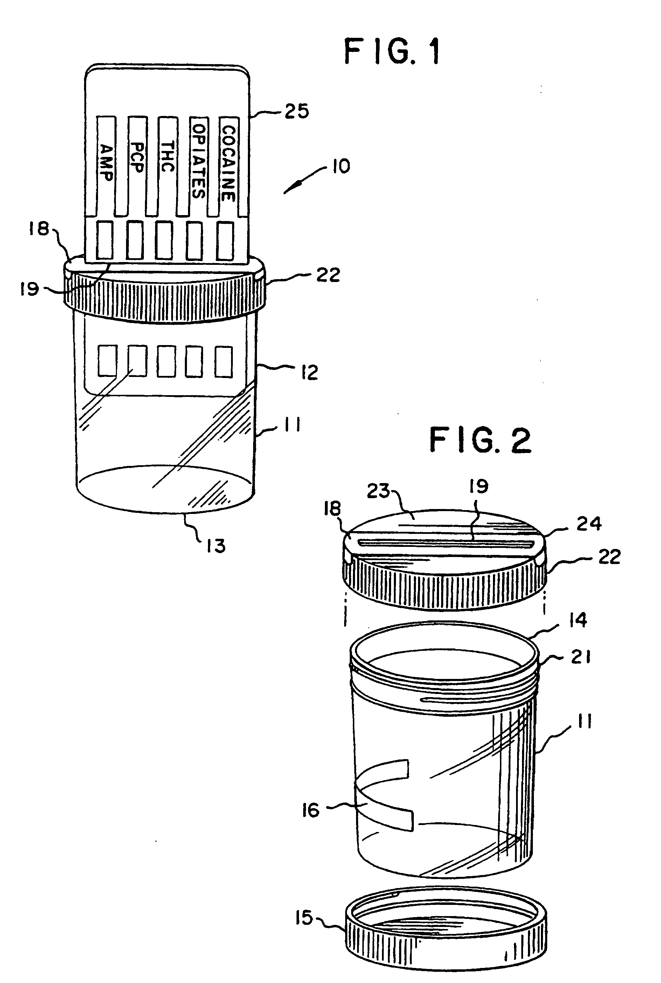

As may be seen in Figs. 1 and 2, a drug abuse test kit according to the present invention is indicated generally at 10 and comprises a cup-like transparent test container or cup 11 having a cylindrical side wall 12, a closed bottom 13 and an open top 14. The cylindrical wall 12 may have a slight taper or be straight.

The open end 14 of the test cup 11 is provided with external threads 21 upon which is seated an outer closure cover or cap 22 provided with corresponding internal threads which are not shown in the drawing. The cover 22 has a circular top surface 23 from the periphery of which depends a cylindrical wall 24 on the inner surface of which there are provided internal threads. The cover surface 23 has a diametrical slit 19 therein shaped to accommodate a test card as will be presently described. There is also provided a solid cover or cap 15 which is similar in size and shape to the cover 22 but is solid or unslit so that the covers 15 and 22 may be interchangeably mounted on the open end 14 of the test cup 11. During shipment, the cover 15 is generally fitted on the bottom of the test cup. A temperature strip 16 is mounted on the bottom side wall of the test cup so as to be responsive to the temperature of the test sample within a test cup.

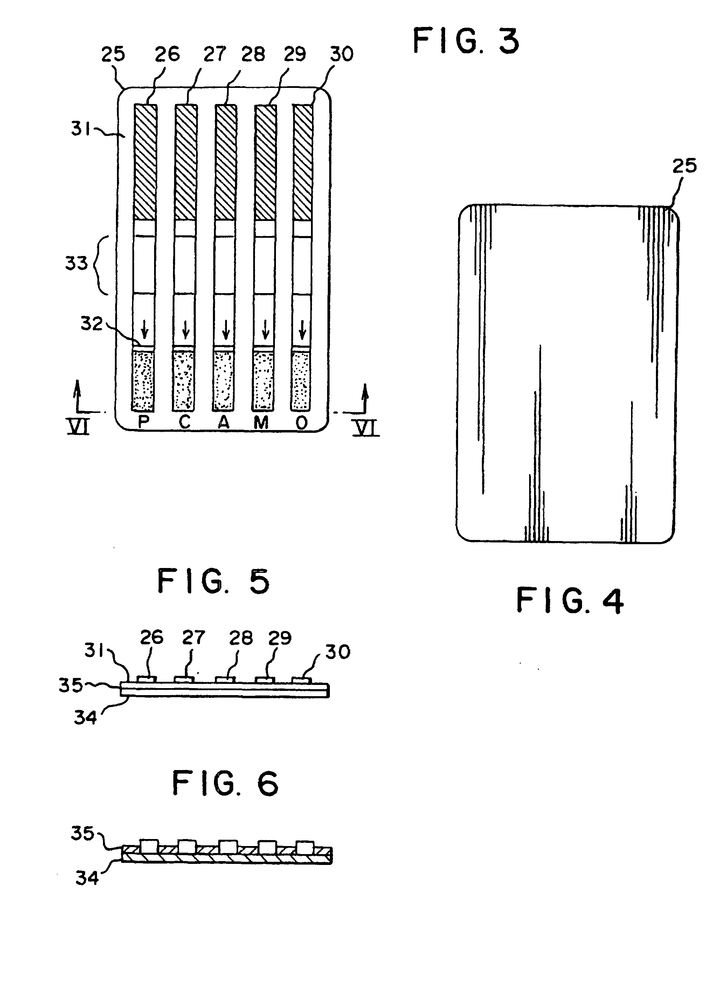

A test card 25 which will indicate the presence or absence of any one of 5 different drugs of abuse is shown in Fig. 1 inserted within the slit 19 in the closure cap 22 and in further detail in Figs. 3-6. The test card is of the multiple drug type in that test strips for five different drugs of abuse are mounted on the test card. The test strips 26-30 are spaced apart in parallel on a test side 31 of the test card. These test strips indicate the presence or absence of the following specific drugs of abuse: PCP, cocaine, amphetamines (AMP), marijuana (THC) and opiates. Test strips 26-30 may be of the type as made by Bionike of South San Francisco, California, Phamatech of San Diego, California and Arista Biological of Bethlehem, Pennsylvania. Such test strips are characterized as immunoassay strips and employ colloidal gold chemistry. Each test strip is submerged up to a maximum line indicated at 32 and the results of the test are read in a test area indicated at 33. A blue line in the test area indicates positive or the presence of the particular drug in the test sample.

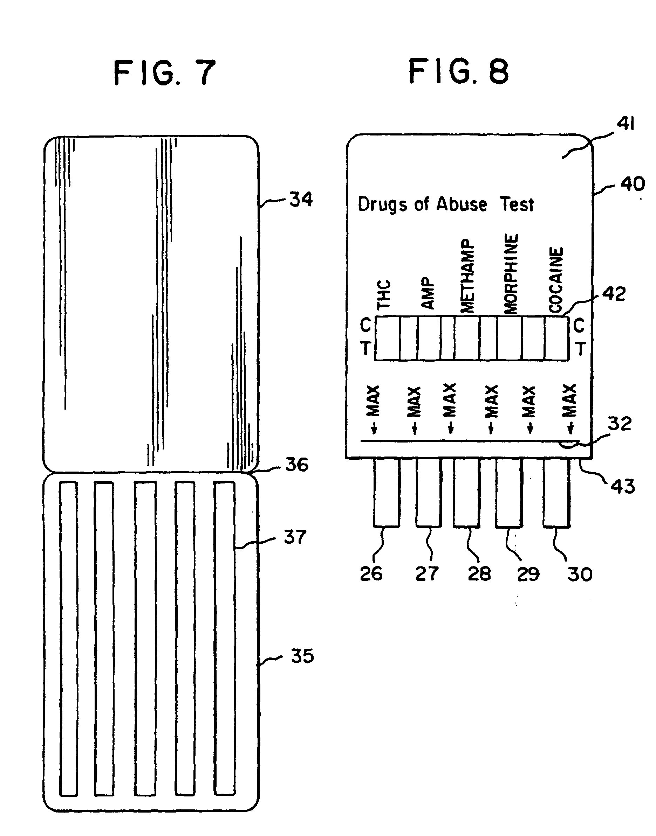

The test strips are actually recessed in slots in the card so that portions of the test strips project above the test surface 31 of the card as may be seen in Fig. 5. The test card may be formed of two plys 34 and 35 as may be seen in Fig. 7 and these plys in turn are formed from a single strip having a bend or fold 36. The ply 35 is formed with a plurality of die cut slots 37 which are shaped and sized to receive each of the test strips. Thus, in the fabrication of a test card, the two portions 34 and 35 are folded over at end 36 and are adhered together. The test strips are then placed into the slots as shown in Fig. 6 and each of the test strips is adhered to the surface of the first portion 34 upon which the second portion 35 has been folded.

It is also within the scope of this invention to make this test card of two separate or individual plys 34 and 35 which are then adhered together and the strips are fixed in the slots as described above.

In order to conduct a drug abuse test utilizing the test card according to the present invention a person being tested must first provide a urine specimen into the transparent test cup 11. The quantity of specimen provided must be such as to permit insertion of the test card up to about the maximum line indicated at 32. It is also possible to provide fill lines on the wall surface of the test container.

The test cup with a sufficient quantity of test specimen therein is then closed by threading the cap 22 on the top of the test cup. The cap 22 is provided with a readily removable adhesive sealing strip 18 which is placed over the slit 19. Thus, when the container with the test specimen is brought to the person conducting the test, the protective strip 18 is removed and the multiple drug test card 25 inserted into the slit so that the bottom of the test card rests upon the bottom of the test cup. Fifteen (15) ml. of specimen will ensure that the specimen does not go above the maximum fill line 32. The test card then remains in place for at least three minutes and the results of the test can be read on each individual test strip through the transparent wall of the container. Thus, if a blue line appears on any one of the test strips, this indicates positive and the presence of that particular drug of abuse in the test specimen. If no such blue line appears then the absence of any of the five drugs of abuse from the specimen is indicated. With such a negative result, the urine sample and the container are discarded.

However, when the results of the test are positive, it is preferable to send the specimen to a certified laboratory for a confirmatory analysis by more specific methods of testing such as gas chromatography or mass spectrometry. In order to ship the sample in the container, the closure 22 is removed and the solid cover 15 is threaded down tightly upon the open end of the container.

In Fig. 8, there is shown at 40 a modification of the test card as described above and is similarly constructed with two plys but is also provided with a third or top ply 41 which is adhered to the two plys and covers the test strips. The third play 41 is provided with an opening 42 through which the test and control lines may be seen. In this modification, those portions of the plys below the maximum fill line 32 are removed such that the test strips 26-30 project beyond a bottom end 43 of the shortened test card. Otherwise, this test card functions in precisely the same manner as described above.

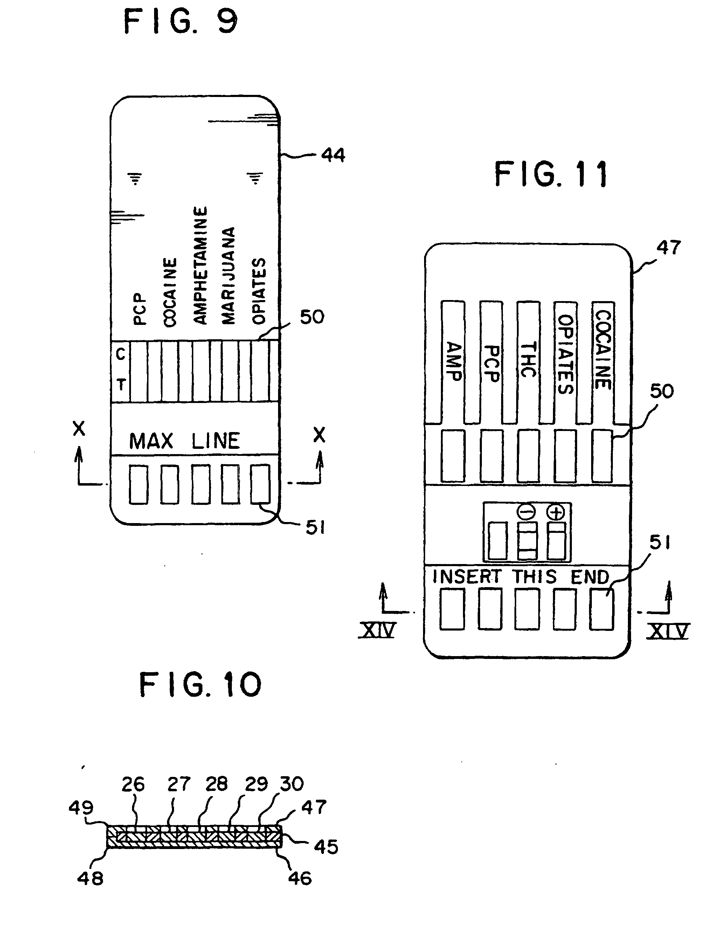

A modification of the test card is shown at 44 in Fig. 9. In this modification, the test strips are covered but the pertinent test and sample portions of the test strips are exposed through openings. The test card 44 comprises a central ply 45 of styrene which has a thickness of 1.25 mm. corresponding to or slightly greater than the thickness of the test strips and slots are provided in the center ply to receive the test strips. The top and bottom faces of the central ply 45 are covered by a bottom ply 46 and a top ply 47 which may be made from a single piece of material double scored at 48 and 49 so as to wrap around the central ply 45 in the manner as shown in Fig. 9. The top and bottom plys may be of a thin vinyl sheet or cardboard coated with plastic. The top ply 47 is provided with a plurality of test windows 50 through which the test results as indicated by the test strips can be seen. At the lower end of the card are provided sample openings 51 through which the liquid test specimen is able to contact the absorbent or sample portions of the test strips.

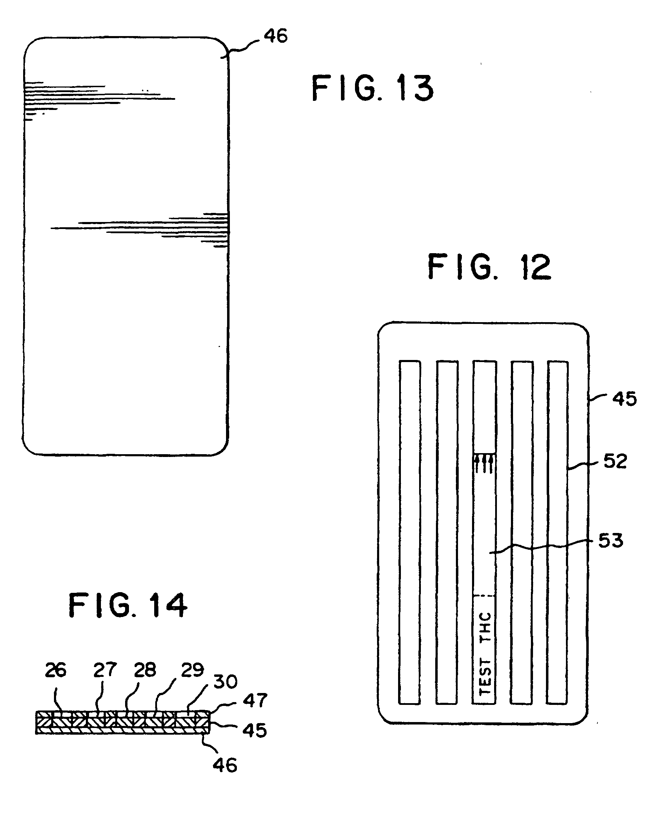

In Figs. 11-14 there is shown a modification of the test card 44 in which the card is made of three separate plys which are then laminated. The bottom and top plys 46 and 47 are made of a thin vinyl sheet having a thickness of 0.33 mm. and the center ply 45 is made of styrene having a thickness of about 1.25 mm. The top ply 47 similarly has the test openings or windows 50 and the sample openings 51 and the bottom ply 46 is solid as shown.

The central ply 45 is provided with a plurality of longitudinally extending slots 52 and a test strip 53 is seated in each of these slots as shown. The test strip generally has a length less than that of the slot 52. In this embodiment, only a single test strip for THC (marijuana) is shown. While this embodiment of the test card has provision for five test strips, it is to be understood that the card can be made in the same manner with less than five strips and even a single strip if so desired. In such a modification, the windows 50 and 51 for the omitted strips are usually solid.

Each of the test strips 26-30 is a one-step immunoassay in which a specially labeled drug, (drug conjugate) competes with drug which may be present in the sample for the limited number of binding sites on an antibody. The test strip consists of a membrane strip onto which a drug conjugate has been immobilized. A colloidal gold-antibody complex is dried at one end of the membrane. In the absence of any drug in the urine sample, the colloidal gold-antibody complex moves with the urine sample by capillary action to contact the immobilized drug conjugate. An antibody-antigen reaction occurs forming a visible line in the test area. The formation of a visible line in the test area occurs when the test is negative for the drug. When a drug is present in the urine sample, the drug or its metabolite will compete with the immobilized drug conjugate in the test area for the limited antibody sites on the colloidal gold-labeled antibody complex. If a sufficient amount of drug is present, it will fill all of the available binding sites, thus preventing attachment of the label antibody to the drug conjugate. An absence of a color line or band in the test area is indicative of a positive result. A control band or line comprised of a different antibody/antigen reaction is present on the membrane strip. The control line is not influenced by the presence or absence of drug in the urine and therefore should be present in all reactions.

In summary, if a single band appears in the control zone and no band appears in the test zone then the results are "positive" which indicates that that particular drug is present above a predetermined level which is usually around 50ng/ml.
If two color bands appear, one in the control region and the other in the test region then the test results are "negative" which indicates that the level of that particular drug is below the predetermined detection of sensitivity.

In the event that there are no distinct color bands visible in both the test zone and the control zone or if there is a visible band in the test zone but not in the control zone, then the result is invalid and retesting of the specimen is recommended with another test card.

The test card can also be used as a carrier or delivery system for a biological detection or monitor device by replacing the drug test strips with strips treated with suitable chemicals so as to be responsive to different and selected biological warfare agents. The strips would then function similarly to drug abuse strips to provide a visual indication of the presence in a predetermined quantity of a specific biological warfare agent or the absence of such an agent.

### INDUSTRIAL APPLICABILITY

Thus it can be seen that the present invention discloses a novel and improved drug abuse test kit which comprises a container for the fluid specimen being tested and a multiple drug test card which is inserted in the specimen within the container and the visual results of the test are read on the test card through the transparent wall of the container. The test card thus comprises a number of individual test strips of the immunoassay type and each strip is responsive or indicative to a particular drug of abuse. The test card may be made of plastic coated cardboard or thin sheets of plastic which are laminated together. This drug abuse test kit enables one to obtain rapidly a visual, qualitative result which is very advantageous for forensic purposes but is not limited to such purposes.

It will be understood that this invention is susceptible to modification in order to adapt it to different usages and conditions, and accordingly, it is desired to comprehend such modifications within this invention as may fall within the scope of the appended claims.

## Claims

1. A drug abuse test kit (10) comprising a cup-like container (11) having an open top end (14) for retaining a fluid sample to be tested, a closure cap (22) seated upon said open top end (14) and a test card (25; 40; 44) having visual indication test means on one side (31) thereof, **characterized in that** the closure cap (22) has a diametrically disposed slit (19) therein, and that the visual indication test means comprise one or more immunoassay visual indication test strips (26-30; 53) with a visual end point to indicate the presence or absence of a drug disposed in parallel on one side (31) of said test card (25; 40; 44), said test card (25; 40; 44) having such a width and thickness that a bottom end of said test card (25; 40; 44) is insertable through said slit (19) to be immersed in a fluid sample retained therein to a predetermined depth, the results of each test strip (26-30; 53) can be seen through a transparent wall (12) of the container (11) without removing the test card (25; 40; 44) from the container (11) to indicate the presence or absence of a particular drug of abuse in said fluid sample.

2. A drug abuse test kit according to claim 1, further comprising a solid second closure cap (15) positionable over the open end (14) of the container instead of said closure cap (22) with a slit (19).

3. A drug abuse test kit according to one of the preceding claims 1-2, further comprising a protective adhesive strip (18) over said slit (19) which is removable prior to testing the specimen within the container.

4. A drug test card for testing of a fluid sample in a container (11) having an open top end (14), the test card (25; 40; 44) comprising a thin flat member having the size and shape of a business card, said thin flat member having a longitudinal dimension extending between top and bottom ends and having visual indication test means on one side (31) thereof, wherein the thin flat member is shaped such that its bottom end is insertable longitudinally into the container (11) to contact a fluid sample therein, and that the visual indication test means comprise one or more immunoassay test strips (26-30; 53) with a visual end point to indicate the presence or absence of a drug, disposed longitudinally in parallel on said first side (31) and having at least portions thereof exposed, each test strip (26-30;53) indicating the presence or absence of a particular drug of abuse and having a sample receiving portion (51) at its bottom end and further having a test portion (33) spaced longitudinally therefrom wherein, in use, the fluid sample moves by capillary action to the test portion (33) at which the presence or absence of the selected drug of abuse in the fluid sample is visually indicated, the bottom end of each said one or more test strips (26-30; 53) being disposed at the bottom end of said thin flat member.

5. A drug test card according to claim 4, wherein a plurality of immunoassay test strips (26-30; 53) are adhered side-by-side in parallel on said first side (31).

6. A drug test card according to claim 4 or claim 5, wherein a test strip (26-30; 53) is recessed in said first side (31).

7. A drug test card according to one of the preceding claims 4-6, wherein a test strip (26-30; 53) is seated in a slot (37; 52) on said first side (31).

8. A drug test card according to one of the preceding claims 4-7, wherein an exposed portion of a test strip is recessed inwardly of said first side surface (31).

9. A drug test card according to one of the preceding claims 4-8, wherein said thin flat member comprises three laminated sheets, one of said sheets defining a backing sheet (46), a second (45) of said sheets having a plurality of parallel slots (52) therein to receive said test strips (26-30; 53) and a third (47) of said sheets having spaced parallel slots therein according with said test strips, said sheets being adhered together such that said first and third sheets (46, 47) sandwich the second sheet (45) there between.

10. A drug test card according to one of the preceding claims 4-9, wherein said thin flat member (45) has front and rear surfaces and a thickness substantially equal to the thickness of said test strips, there being slots in said thin flat member to receive said test strips therein, a second thin flat member (46) adhered to said rear surface of said thin flat member (45), a third thin flat member (47) adhered to the front surface of said thin flat member (45), there being openings (50, 51) in said third thin flat member (47) to expose the sample and test portions of each of said test strips.

11. A drug test card according to claim 10, wherein said second and third thin flat members (46, 47) comprise a single sheet of material having a fold therein and folded around said first thin flat member (45).

12. A drug test card according to claim 10, wherein said second and third thin flat members (46, 47) are thinner than said first thin flat member (45).

13. A drug test card according to one of the preceding claims 4-12, wherein said test card (40) has a bottom end (43) and said test strips (26-30) have ends projecting outwardly of said bottom end (43).

14. A drug test card according to claim 13, further comprising a cover sheet (41) covering said first side of said thin flat member and said test strips, there being an opening (42) in said cover sheet to expose portions of said test strips.

## Patentansprüche

1. Nachweisset (10) zum Nachweisen von Drogenmißbrauch, welches einen becherförmigen Behälter (11) mit einem offenen oberen Ende (14) zur Aufnahme einer zu untersuchenden Flüssigkeitsprobe, eine auf das offene obere Ende (14) aufgesetzte Verschlußkappe (22) und eine Nachweiskarte (25; 40; 44) mit einer Einrichtung zum Nachweis durch Sichtanzeige auf einer (31) ihrer Seiten aufweist, **dadurch gekennzeichnet, daß** die Verschlußkappe (22) einen diametral darin vorgesehenen Schlitz (19) aufweist und daß die Einrichtung zum Nachweis durch Sichtanzeige einen oder mehrere parallel auf einer Seite (31) der Nachweiskarte (25; 40; 44) angeordneten Teststreifen (26 - 30; 53) für die Immunanalyse mit Sichtanzeige mit einem sichtbaren Endpunkt zur Anzeige aufweist, ob eine Droge bzw. ein Medikament vorhanden oder nicht vorhanden ist, wobei die Nachweiskarte (25; 40; 44) eine solche Breite und Stärke aufweist, daß ein unteres Ende der Nachweiskarte (25; 40; 44) durch den Schlitz (19) in der Weise einführbar ist, daß es in eine darin enthaltene Flüssigkeitsprobe bis in eine vorgegebene Tiefe eingetaucht ist, und wobei die Ergebnisse an jedem Teststreifen (26 - 30; 53) ohne Entfernen der Nachweiskarte (25; 40; 44) aus dem Behälter (11) durch eine durchsichtige Wandung (12) des Behälters (11) hindurch in der Weise sichtbar sind, daß das Vorhandensein oder Nichtvorhandensein einer speziellen mißbräuchlich eingenommenen Droge bzw. eines speziellen mißbräuchlich eingenommenen Medikaments in der Flüssigkeitsprobe angezeigt wird.

2. Nachweisset zum Nachweisen von Drogenmißbrauch nach Anspruch 1, welches des weiteren eine massive zweite Verschlußkappe (15) aufweist, die über dem offenen Ende (14) des Behälters anstelle der Verschlußkappe (22) mit einem Schlitz (19) positionierbar ist.

3. Nachweisset zum Nachweisen von Drogenmißbrauch nach einem der vorhergehenden Ansprüche 1 - 2, welches außerdem einen Klebstreifen (18) zum Schutz über dem Schlitz (19) aufweist, der vor dem Testen der im Inneren des Behälters befindlichen Probe abziehbar ist.

4. Nachweiskarte zum Nachweisen von Drogen bzw. Medikamenten zum Testen einer Flüssigkeitsprobe in einem Behälter (11) mit einem oberen offenen Ende (14), wobei die Nachweiskarte (25; 40; 44) ein dünnes flaches Teil in der Größe und Form einer Visitenkarte aufweist und wobei das dünne flache Teil in Längsrichtung eine Erstreckung aufweist, die sich zwischen dem oberen und unteren Ende erstreckt und auf einer (31) seiner Seiten eine Einrichtung zum Nachweis durch Sichtanzeige aufweist, bei welcher das dünne flache Teil in der Weise geformt ist, daß sein unteres Ende in Längsrichtung so in den Behälter (11) einsetzbar ist, daß es mit einer darin enthaltenen Flüssigkeitsprobe in Berührung kommt und daß die Einrichtung zum Nachweis durch Sichtanzeige einen oder mehrere Teststreifen (26 - 30; 53) zur Immunanalyse mit einem sichtbaren Endpunkt aufweist, welcher anzeigt, ob eine Droge bzw. ein Medikament vorhanden oder nicht vorhanden ist, welche in Längsrichtung parallel auf der ersten Seite (31) angeordnet sind und bei denen zumindest Abschnitte freiliegen, wobei jeder Teststreifen (26 - 30; 53) das Vorhandensein oder Nichtvorhandensein einer speziellen mißbräuchlich eingenommenen Droge bzw. eines speziellen mißbräuchlich eingenommenen Medikaments anzeigt, bei welcher am unteren Ende ein Aufnahmeabschnitt (51) zur Aufnahme der Probe vorgesehen ist und in Längsrichtung im Abstand hiervon ein Nachweisabschnitt (33) ausgebildet ist und wobei sich im Einsatz die Flüssigkeitsprobe durch Kapillarwirkung zu dem Nachweisabschnitt (33) bewegt, an welchem das Vorhandensein oder Nichtvorhandensein der ausgewählten mißbräuchlich eingenommenen Droge bzw. des Medikaments in der Flüssigkeitsprobe sichtbar angezeigt wird, und daß das untere Ende des einen bzw. jedes der Teststreifen (26 - 30; 53) am unteren Ende des dünnen flachen Teils angeordnet ist.

5. Nachweiskarte zum Nachweisen von Drogen bzw. Medikamenten nach Anspruch 4, bei welcher eine Vielzahl von Teststreifen (26 - 30; 53) zur Immunanalyse nebeneinander parallel auf der ersten Seite (31) angeklebt ist.

6. Nachweiskarte zum Nachweisen von Drogen bzw. Medikamenten nach Anspruch 4 oder Anspruch 5, bei welcher ein Teststreifen (26 - 30; 53) eine Vertiefung in der ersten Seite (31) aufweist.

7. Nachweiskarte zum Nachweisen von Drogen bzw. Medikamenten nach einem der vorhergehenden Ansprüche 4 - 6, bei welcher ein Teststreifen (26 - 30; 53) in einem Schlitz (37; 52) auf der ersten Seite (31) sitzt.

8. Nachweiskarte zum Nachweisen von Drogen bzw. Medikamenten nach einem der vorhergehenden Ansprüche 4 - 7, bei welcher ein freiliegender Abschnitt eines Teststreifens von der ersten Seitenfläche (31) aus nach innen vertieft ist.

9. Nachweiskarte zum Nachweisen von Drogen bzw. Medikamenten nach einem der vorhergehenden Ansprüche 4 - 8, bei welcher das dünne flache Teil drei aufeinander laminierte Folien aufweist, wobei eine der Folien eine Trägerfolie (46) definiert, eine zweite (45) der Folien eine Vielzahl von darin ausgebildeten parallelen Schlitzen (52) zur Aufnahme der Teststreifen (26 - 30; 53) aufweist und in einer dritten (47) der Folien parallel im Abstand voneinander vorgesehene Schlitze in Entsprechung zu den Teststreifen ausgebildet sind, wobei die Folien in der Weise aneinandergeklebt sind, daß die erste und die dritte Folie (46, 47) die zweite Folie (45) zwischen sich einschließen.

10. Nachweiskarte zum Nachweisen von Drogen bzw. Medikamenten nach einem der vorhergehenden Ansprüche 4 - 9, bei welcher das dünne flache Teil (45) eine Vorderfläche und eine rückwärtige Fläche und eine Stärke aufweist, die im wesentlichen gleich der Stärke der Teststreifen ist, wobei in dem dünnen flachen Teil Schlitze zur Aufnahme der Teststreifen vorgesehen sind, ein zweites dünnes flaches Teil (45) an der rückwärtigen Fläche des dünnen flachen Teils (46) angeklebt ist, ein drittes dünnes flaches Teil (47) an der Vorderfläche des dünnen flachen Teils (45) angeklebt ist, und in dem dritten dünnen flachen Teil (47) Öffnungen (50, 51) in der Weise ausgebildet sind, daß sie die Probe und Testabschnitte jedes der Teststreifen freigeben.

11. Nachweiskarte zum Nachweisen von Drogen bzw. Medikamenten nach Anspruch 10, bei welcher das zweite und das dritte dünne flache Teil (46, 47) jeweils eine einzige Materiallage mit einer darin ausgebildeten Falte aufweist, welche um das erste dünne flache Teil geschlagen ist.

12. Nachweiskarte zum Nachweisen von Drogen bzw. Medikamenten nach Anspruch 10, bei welcher das zweite und das dritte dünne flache Teil (46, 47) jeweils dünner als das erste dünne flache Teil (45) ist.

13. Nachweiskarte zum Nachweisen von Drogen bzw. Medikamenten nach einem der vorhergehenden Ansprüche 4 - 12, bei welcher die Nachweiskarte (40) ein unteres Ende (43) aufweist und die Teststreifen (26 - 30) Enden besitzen, die sich über das untere Ende (43) hinaus nach außen erstrecken.

14. Nachweiskarte zum Nachweisen von Drogen bzw. Medikamenten nach Anspruch 13, welche außerdem eine Abdeckfolie (41 ) aufweist, welche die erste Seite des dünnen flachen Teils und die Teststreifen abdeckt, wobei eine Öffnung (42) in der Abdeckfolie vorgesehen ist, welche Abschnitte der Teststreifen freigibt.

## Revendications

1. Kit de test de toxicomanie (10) comportant un conteneur en forme de coupelle (11) ayant une extrémité supérieure ouverte (14) pour retenir un échantillon de fluide à tester, un capuchon de fermeture (22) en appui sur ladite extrémité supérieure ouverte (14) et une carte de test (25 ; 40 ; 44) ayant des moyens d'indication visuelle de test sur un premier côté (31), **caractérisé en ce que** le capuchon de fermeture (22) a une fente (19) disposée suivant un diamètre, et **en ce que** les moyens d'indication visuelle de test comportent une ou plusieurs bandes d'indication visuelle de test d'essai immunologique (26 à 30 ; 53) ayant un emplacement d'extrémité visuelle pour indiquer la présence ou l'absence d'une drogue, disposées parallèlement sur un premier côté (31) de ladite carte de test (25 ; 40 ; 44), ladite carte de test (25 ; 40 ; 44) ayant une largeur et une épaisseur telles qu'une extrémité inférieure de ladite carte de test (25 ; 40 ; 44) peut être insérée à travers ladite fente (19) pour être immergée dans un échantillon de fluide qui y est retenu jusqu'à une profondeur prédéterminée, les résultats de chaque bande de test (26 à 30 ; 53) pouvant être vus à travers une paroi transparente (12) du conteneur (11) sans enlever la carte de test (25 ; 40 ; 44) du conteneur (11) pour indiquer la présence ou l'absence d'une drogue particulière dans ledit échantillon de fluide.

2. Kit de test de toxicomanie selon la revendication 1, comportant de plus un second capuchon de fermeture plein (15) pouvant être positionné au-dessus de l'extrémité ouverte (14) du conteneur à la place dudit capuchon de fermeture (22) ayant une fente (19).

3. Kit de test de toxicomanie selon la revendication 1 ou 2, comportant de plus une bande d'adhésif de protection (18) située sur ladite fente (19), qui peut être enlevée avant de tester le spécimen situé dans le conteneur.

4. Carte de test de toxicomanie destinée à tester un échantillon de fluide situé dans un conteneur (11) ayant une extrémité supérieure ouverte (14), la carte de test (25, 40, 44) comportant un élément plat mince ayant la dimension et la forme d'une carte de visite, ledit élément plat mince ayant une dimension longitudinale s'étendant entre des extrémités supérieure et inférieure et ayant des moyens d'in- dication visuelle de test sur un premier côté (31, l'élément plat mince étant mis en forme de telle sorte que son extrémité inférieure puisse être insérée longitudinalement dans le conteneur (11) pour venir en contact avec un échantillon de fluide qui est situé dedans, et en ce que les moyens d'indication visuelle de test comportent une ou plusieurs bandes de test d'essai immunologique (26 à 30 ; 53) ayant un emplacement d'extrémité visuelle pour indiquer la présence ou l'absence d'une drogue, disposées longitudinalement parallèlement sur ledit premier côté (31) et ayant au moins des parties exposées, chaque bande de test (26 à 30 ; 53) indiquant la présence ou l'absence d'une drogue particulière et ayant une partie de réception d'échantillon (51) à son extrémité inférieure et ayant de plus une partie de test (33) espacée longitudinalement à partir de celle-ci, dans laquelle, en utilisation, l'échantillon de fluide se déplace par action de capillarité vers la partie de test (33) au niveau de laquelle la présence ou l'absence de la drogue sélectionnée, dans l'échantillon de fluide, est visuellement indiquée, l'extrémité inférieure de chacune desdites une ou plusieurs bandes de test (26 à 30 ; 53) étant disposée à l'extrémité inférieure dudit élément plat mince.

5. Carte de test de toxicomanie selon la revendication 4, dans laquelle une pluralité de bandes de test d'essai immunologique (26 à 30 ; 53) sont collées côte à côte parallèlement sur ledit premier côté (31).

6. Carte de test de toxicomanie selon la revendication 4 ou 5, dans laquelle une bande de test (26 à 30 ; 53) est creusée dans ledit premier côté (31).

7. Carte de test de toxicomanie selon l'une quelconque des revendications 4 à 6, dans laquelle une bande de test (26 à 30 ; 53) est appuyée dans une fente (37 ; 52) existant sur ledit premier côté (31).

8. Carte de test de toxicomanie selon l'une quelconque des revendications 4 à 7, dans laquelle une partie exposée d'une bande de test est creusée vers l'intérieur de ladite surface du premier côté (31).

9. Carte de test de toxicomanie selon l'une quelconque des revendications 4 à 8, dans laquelle ledit élément mince plat comporte trois feuilles stratifiées, une première desdites feuilles définissant une feuille de support (46), une deuxième (45) desdites feuilles ayant une pluralité de fentes parallèles (52) agencées pour recevoir lesdites bandes de test (26 à 30 ; 53) et une troisième (47) desdites feuilles ayant des fentes parallèles espacées agencées conformément auxdites bandes de test, lesdites bandes étant collées ensemble de telle sorte que lesdites première et troisième feuilles (46, 47) enserrent entre elles la deuxième feuille (45).

10. Carte de test de toxicomanie selon l'une quelconque des revendications 4 à 9, dans laquelle ledit élément plat mince (45) comporte des surfaces avant et arrière et une épaisseur sensiblement égale à l'épaisseur desdites bandes de test, des fentes existant dans ledit élément plat mince pour y recevoir lesdites bandes de test, un deuxième élément plat mince (46) est collé sur ladite surface arrière dudit élément plat mince (45), un troisième élément plat mince (47) est collé sur la surface avant dudit élément plat mince (45), des ouvertures (50, 51) existant dans ledit troisième élément plat mince (47) pour exposer l'échantillon et les parties de test de chacune desdites bandes de test.

11. Carte de test de toxicomanie selon la revendication 10, dans laquelle lesdits deuxième et troisième éléments plats minces (46, 47) sont constitués d'une feuille unique de matériau ayant un pli et pliée autour dudit premier élément plat mince (45).

12. Carte de test de toxicomanie selon la revendication 10, dans laquelle lesdits deuxième et troisième éléments plats minces (46, 47) sont plus minces que ledit premier élément plat mince (45).

13. Carte de test de toxicomanie selon l'une quelconque des revendications 4 à 12, dans laquelle ladite carte de test (40) a une extrémité inférieure (43) et lesdites bandes de test (26 à 30) ont des extrémités faisant saillie vers l'extérieur de ladite extrémité inférieure (43).

14. Carte de test de toxicomanie selon la revendication 13, comportant de plus une feuille de recouvrement (41) recouvrant ledit premier côté dudit élément plat mince et lesdites bandes de test, une ouverture (42) existant dans ladite feuille de recouvrement pour exposer des parties desdites bandes de test.
